# EUROPEAN PATENT SPECIFICATION

(11) **EP 2 523 636 B1**
(45) Date of publication and mention of the grant of the patent: **01.07.2015**
(21) Application number: 10809251.1
(22) Date of filing: 23.12.2010
(51) Int. Cl.: A61F 2/58

(54) **MODULAR HUMAN HAND PROSTHESIS WITH MODULAR, MECHANICALLY INDEPENDENT FINGER MODULES**
MODULARE MENSCHLICHE HANDPROTHESE MIT MODULAREN MECHANISCH UNABHÄNGIGEN FINGERMODULEN
PROTHÈSE MODULAIRE DE MAIN HUMAINE AVEC MODULES MODULAIRES DE DOIGTS MÉCANIQUEMENT INDÉPENDANTS

(30) Priority: 14.01.2010 PL 39018810
(43) Date of publication of application: 21.11.2012
(73) Proprietor: Tumbaga Sp. z.o.o., 30-348 Krakow (PL)
(72) Inventor: JOPEK, Wojciech, PL-59-900 Zgorzelec (PL); TUROW, Michal, PL-50-107 Wroclaw (PL); WASIELEWSKI, Michal, PL-05-120 Legionowo (PL); KUCHARSKI, Michal, PL-05-509 Jozefoslaw (PL)
(74) Representative: Drelichowski, Henryk
(86) International application number: PCT/PL2010/000124
(87) International publication number: WO 2011/087382

(56) References cited:
- WO-A1-95/24875
- WO-A1-2007/063266
- WO-A2-2008/098072
- GUO G ET AL: "OPTIMAL DESIGN OF A SIX-BAR LINKAGE WITH ONE DEGREE OF FREEDOM FOR AN ANTHROPOMORPHIC THREE-JOINTED FINGER MECHANISM", PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, vol. 207, no. H03, PART H, 1 January 1993 (1993-01-01), pages 185-190, XP000414518, ISSN: 0954-4119

## Description

The invention is a modular human hand prosthesis with modular, mechanically independent finger modules, which may be applied in prosthetics as a replacement of a single finger, a group of fingers or a whole hand or arm.

Hand prosthesis, known from the Polish patent application no. P.372638, consists of a shaft axis with grasping fingers, which is twisted relative to the shaft axis with a prehensile thumb, and the thumb is rotary embedded with a change of twist angle in the yoke, while the shafts with fingers are connected with each other by means of a spherical toothed gear, and the drive shaft is connected by the conical toothed gear with a shaft with grasping fingers, and with the yoke by means of a cylindrical gear with a yoke.

Hand prosthesis, known from Polish patent application no. P.372639, consists of a shaft axis with grasping fingers, which is twisted to the shaft axis with prehensile thumb, and the thumb is rotary embedded with the change of twist angle in the yoke, while the shafts with fingers are connected with each other by means of a rotating link and a connector ended with ball-and-socket joints forming a spatial pentagon joint. At the same time, the yoke is connected via a cylindrical toothed gear with drive shaft, the shaft with gripping fingers through a conical toothed gear with the drive shaft, and the shaft along with the prehensile finger is connected with the drive shaft via a rotating element and a conical toothed gear.

The hand prosthesis, known from the Polish patent application no. P.379607, consists of a shaft axis with grasping fingers, which is twisted relative to the shaft axis with a prehensile finger embedded by means of a swivel with movement lock in the yoke, which enables a change of the twist angle. Shafts with fingers perform four types of movements because they are interconnected by a separable cylindrical toothed gear controlled by a disengaging clutch and two permanent conical toothed gears. The drive shaft is connected to the intermediate shaft with two disengaging cylindrical toothed gears controlled by disengaging clutches. The finger prosthesis of the upper limb, known from the Polish patent application no P.387380, has a linear drive, fixed at the finger base, equipped with an articulated joint connected through the first rod, the second articulated joint, the proximal phalanx and the fifth articulated joint with a distal phalange and, at the same time, the second joint is connected rigidly to the proximal phalanx rod bearing and rotatably with the third articulated joint, which is positioned at the finger base bearing. The fifth articulated joint is connected through the distal phalanx rod, the sixth joint and second rod with the fourth joint mounted at the finger base. The joints are located in the articulated pentagon corners performing movement resembling the movement of a finger.

The prosthesis, known from the patent application No. WO 9524875, consists of at least one mechanically efficient finger segment, with at least one finger segment positioned tangentially to a worm wheel fixed in the body of the prosthesis and at the same time bearinged rotationally in the worm wheel axis. The finger segment has a driving engine with a worm located along the finger segment and simultaneously coupled by means of the worm teeth to the worm wheel. While using the prosthesis, the finger segment moves around the worm wheel to or from another finger segment and / or to the natural finger opening in order to close and open the hand grip during the drive motor operation.

The prosthesis, known from the patent application No. WO 2007063266, consists of at least one mechanically efficient finger segment, which is positioned tangentially to a worm wheel fixed in the bracket segment of the prosthesis and at the same time bearinged rotationally in the worm wheel axis. The finger segment has a drive motor used to drive the worm. The worm is coupled with the worm wheel in a way that when the engine is turned on, the prosthesis is used, the finger segment moves around the worm wheel, which is located outside the finger segment.

At the moment, the development of a prosthesis allowing to grasp objects of different shapes, sizes and dimensions and weights with adjustable strength is a considerable problem. An additional requirement, considering the support for independent movement of individual fingers in a manner similar to the natural movement of the fingers, while keeping the weight and dimensions of the device similar to those typical of the human hand, makes this task even more complicated. Another problem is the development of a prosthesis which will have a modular design characterized by independent mechanical fingers of at least one independent and one dependent degree of freedom, and of a drive system located inside the finger in such a way as to allow the device to replace individual lost fingers as well as an entire arm and/or hand.

The most popular solution on the market for hand prosthetics generally does not have a modular design and only enables a simple hand opening and closing movement and wrist rotation of the hand. The conventional approach is characterized by using a single drive system that drives all the fingers simultaneously. The drive systems of other solutions that are available, whose authors decided to use independently driven fingers, have been developed in such a way that a part of the system together with the motor is located inside a finger. However, unfortunately, the rest of the system is located within the metacarpus. Consequently, it limits the possibility of using the device in case of the absence of a single finger or a group of fingers.

The invention of the modular hand prosthesis described below, with modular, mechanically independent finger modules, solves at least some of the aforementioned problems by placing the entire single finger drive system inside the finger and using the additional space available in the distal phalanx of the prosthesis.

The essence of the invention lies in the fact that it has at least one mechanically independent finger module which in particular consists of a fixed base, a finger proximal segment, a finger distal segment, a rod and a rotational drive, while the finger base equipped with a rotating proximal joint of the rod and a rotating proximal joint of a finger proximal segment is connected via a rod proximal joint, the rod, a rod distal joint and, simultaneously, via the proximal joint of the finger proximal segment, the finger proximal segment, the distal joint of the finger proximal segment with the finger distal segment, and the distal joint of the finger proximal segment constitutes the permanent centre of the rotational drive.

A finger is composed of at least two segments, of which the finger proximal segment is applied as the proximal phalanx and the finger distal segment is used as the distal phalanx. Preferably, in particular an electric motor permanently coupled with a reduction gear, which is connected with a worm gear consisting of a worm and worm wheel, is used as the rotational drive. The electric motor together with the reduction gear is fixed in the finger proximal segment in such a way that the reduction gear output shaft is facing the finger distal segment. In turn, the worm wheel is fixed to the finger distal segment, while the worm, which is geared with it, is mounted on the reduction gear output shaft. The electric motor with a reduction gear is located along the proximal section of the finger while the motor axis is inclined from the geometrical axis of the finger proximal segment in a range of +-30[deg.].

Preferably, when the motor is supplied with power, the worm mounted on the engine output shaft rotates the worm wheel and the associated finger distal segment against the finger proximal segment and particularly against the distal joint of the finger proximal segment.

Preferably, when the finger distal segment moves, it causes rotational movement of the distal segment of the proximal finger in relation to the joint of the distal rod and the rod in relation to the joint of the proximal rod, causing rotational movement of the finger proximal segment in relation to the proximal segment joint of the of the proximal finger of the finger base.

Preferably, when the motor is powered, the finger proximal segment and the finger distal segment perform, relative to the finger base, a complex movement in such a way that the finger distal segment approaches or moves away from the finger base and the finger proximal segment performs a rotational movement relative to the proximal joint of the finger proximal segment of the finger base, causing the closing or opening of the finger module.

Preferably, the rotational drive's function is performed by the electric, pneumatic or hydraulic motor which, through a single gear, a set of gears or without a gear, enables the movement of the finger module.

Preferably, the mechanical system causing the finger distal segment rotational movement is in particular the mechanical and/or hydraulic and or pneumatic gear. Preferably, the ratio of the horizontal part distance of the finger distal segment comprising the distal joint of the finger proximal segment and the rod distal joint to the part perpendicular to it is 0.24a:a, where "a" is the dimension characteristic for the finger distal segment while the ratio of distance between the distal joint of the finger proximal segment and the rod distal joint to the part of the finger distal segment perpendicular to the horizontal part containing the distal joint of the finger proximal segment and the rod distal joint is 0.07 a.

Preferably, the lines formed by the rod proximal joint and the proximal joint of the finger proximal segment and the distal joint of the finger proximal segment and the rod distal joint are parallel to one another if the mechanically independent finger module is in its initial position.

Preferably, the ratio of the distance between the rod proximal joint and the proximal joint of the finger proximal segment to the vertical distance between the proximal joint of the finger proximal segment and the rod distal joint is 0.32 b:b, where b is the characteristic dimension for the finger proximal segment.

Preferably, the ratio of the horizontal distance of the proximal joint of the finger proximal segment from the finger distal segment part perpendicular to the horizontal part containing the distal joint of the finger proximal segment and the rod distal joint, to the vertical distance between the proximal joint of the finger proximal segment and the rod distal joint is 0.1 b.b.

Preferably, the ratio of the characteristic dimension 'a' of the finger distal segment to the characteristic dimension 'b' of the finger proximal segment is 0.9.

Preferably, according to the invention, the device has a single, mechanically independent finger module together with elements connecting the finger module basis with the patient's body, replacing a patient's single lost finger.

Preferably, according to the invention, there are up to four mechanically independent finger modules with the load bearing structure and/or structures connecting the base of all modules or groups of modules, replacing the lost fingers group.

Preferably, according to the invention, the device has five mechanically independent finger modules with the load bearing structure connecting the bases of all modules, and forms a structure that replaces all the lost fingers with or without replacing the hand.

Preferably, according to the invention, there are additional components and/or load bearing structures especially in the form of wrist connectors, wrist, fastening systems in case of upper limb short amputation, belts, harnesses, and other elements performing the cosmetic and or protective function in relation to the mentioned additional components and/or load bearing.

Preferably, the device is controlled by digital and/or analog processed electromyography signals (EMG) and/or mechanomyographic signals (MMG), in particular acousticmyographic and/or accelleromyographic signals, and other biological signals including in particular electrooculographic (EOG) and electroencephalographic (EEG) signals.

Preferably, according to the invention, the device has an outer shell and/or layers that perform an aesthetic and/or protective function against external factors.

The device has a modular design based on fitting a fully functional drive system inside a single finger module, making it fully mechanically independent. The kinematic structure and the type of mechanical gear used mean that the size of the device and its weight depends almost exclusively on the desired strength generated by a grasping finger. In addition, a small number of elements in the kinematic chain of a single mechanically independent finger module significantly increase the device reliability and technological complexity.

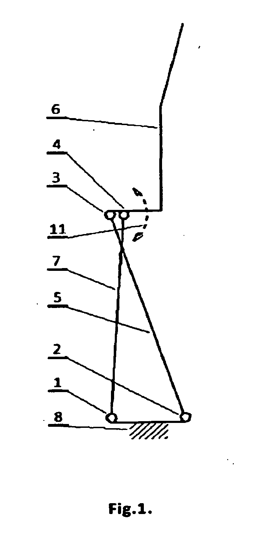

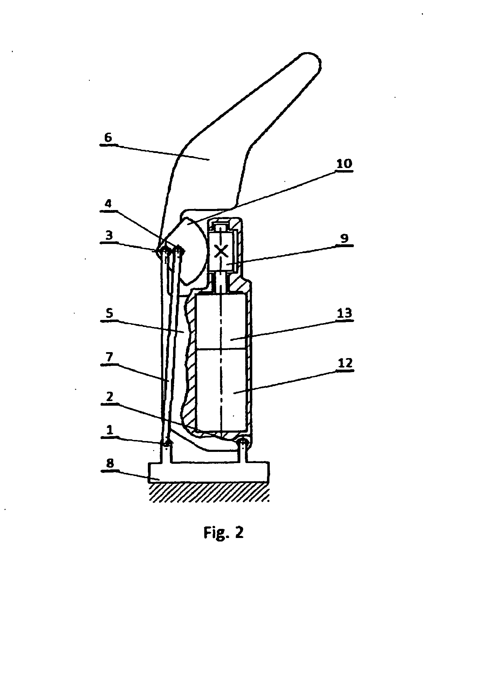

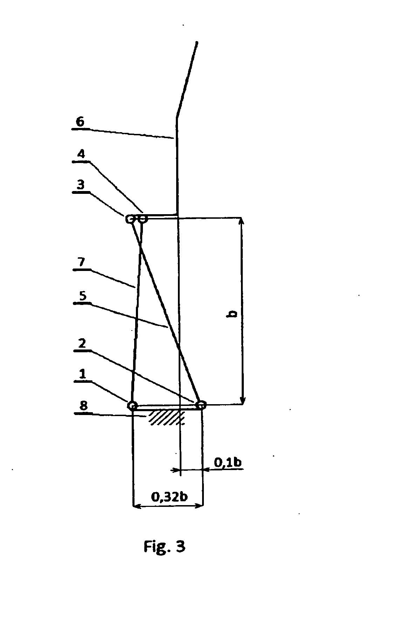

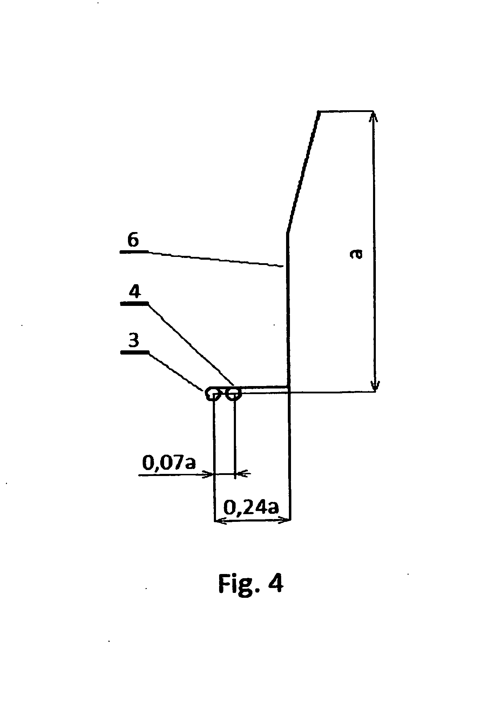

The invention in the form of a sample is shown in the drawing, in which fig. 1 shows the kinematic diagram of the mechanically independent finger module, fig. 2 a sample of the mechanically independent finger module, fig. 3 a kinematic diagram of the mechanically independent finger module with marked geometrical dependence describing the mutual position of the finger distal segment 6 relative to the finger proximal segment 5 and the values characterizing the finger proximal segment 5 and the finger basis 8, fig. 4 a kinematic diagram of the mechanically independent finger module with selected geometrical dependences describing the finger distal segment 6.

### Example 1

The modular human hand prosthesis with modular, mechanically independent finger modules, has a single mechanically independent finger module together with elements connecting the base 8 of the finger module with the patient's body, which replaces a single lost finger of the patient, consisting of, in particular, a fixed finger base 8, the finger proximal segment 5, the finger distal segment 6, the rod 7 and the rotational drive 11, wherein the finger base 8, equipped with the rod rotational proximal joint 1 and the rotational joint of the finger proximal segment 2, is connected via the rod proximal joint 1, the rod 7, the rod distal segment 4 and simultaneously via the proximal joint of the finger proximal segment 2, the finger proximal segment 5, the distal joint of the finger proximal segment 3 with the finger distal segment 6, wherein the distal joint of the finger proximal segment 3 is the rotational permanent centre of the drive 11. The finger proximal segment 5 is used as the proximal phalanx and the finger distal segment 6 - as the distal phalanx. The rotational drive 11 is provided by the electrical motor 12 coupled permanently with reduction gear 13, which is connected to worm gear 9, 10. In the finger proximal segment 5, the electric motor 12 is fixed together with reduction gear 13 in such a way that the reduction gear output shaft is facing the finger distal segment 6. The worm wheel 10, which is connected permanently with the finger distal segment 6, is geared with the worm 9, mounted on the reduction gear output shaft 13. The electric motor 12, together with the reduction gear 13, is located along the finger proximal segment 5 and the motor axis 12 is inclined from the geometrical axis with the axis of the finger proximal segment 5 in the range of +- 30[deg.]. The ratio of the horizontal part distance of the finger distal segment 6 comprising joints 3 and 4 to the part perpendicular to it is 0.24a:a, where a is the dimension characteristic of the finger distal segment 6 while the ratio of distance between joints 3 and 4 to the part of the finger distal segment 6 perpendicular to the horizontal part containing joints 3 and 4 is 0.07a. The lines formed by joints 1 and 2 as well as joints 3 and 4 are parallel to each another if the mechanically independent finger module is in its initial position. The ratio of the distance between joints 1 and 2 to the vertical distance between joints 2 and 4 is 0.32b:b, where b is the dimension characteristic of the finger proximal segment 5 while the ratio of the horizontal distance of the joint 2 from the part of the finger distal segment 6 perpendicular to the horizontal part containing joints 3 and 4 to the vertical distance between joints 2 and 4 is 0.1b:b. The ratio of the characteristic dimension 'a' of the finger distal segment 6 to the characteristic dimension 'b' of the finger proximal segment 5 is 0.9.

The device offers the possibility to use additional construction components and/or load bearing especially in the form of wrist connectors, wrist, fastening systems in case of upper limb short amputation, belts, harnesses, and other elements serving as cosmetic and/or protective function in relation to the mentioned additional components and/or load bearing. Moreover, the prosthesis as invented is controlled by digital and or analog processed electromyography signals (EMG) and/or mechanomyographic signals (MMG), in particular acousticmyographic and/or accelleromyographic signals, and other biological signals including in particular electrooculographic (EOG) and electroencephalographic (EEG) signals. The device may also be equipped with an outer shell and/or having as an aesthetic and/or protective function against external factors.

Electric motor 12 is responsible for forcing the movement of the mechanically independent prosthesis finger module. When powered, the worm 9 mounted on the motor output shaft 12 causes the worm wheel rotation 10 and the related finger distal segment 6 in relation to the finger proximal segment 5 and in particular in relation to the distal joint of the finger proximal segment 3. Then, when the finger distal segment 6 moves, the rotational movement of the distal joint of the finger proximal segment 3 against the rod distal joint 4 and the rod 7 in relation to the rod proximal joint 1 takes place, causing rotational movement of the finger proximal segment 5 in relation to the proximal joint of the finger proximal segment 2 of the finger base 8. Therefore, when the motor 12 is powered, the finger proximal segment 5 and the finger distal segment 6 perform a complex movement against the finger base 8 in such a way that the finger distal segment 6 approaches or moves away from the finger base 8 and from the finger proximal segment 5, and the proximal segment performs rotational movement relative to the proximal joint of the finger proximal segment 2 of the finger base 8 causing the finger module to open or close.

### Example 2

The modular human hand prosthesis with modular, mechanically independent finger modules as in example 1 except that the role of the rotational drive 11 is performed by an electrical, pneumatic or hydraulic motor which, through a single gear, set of gears or without any gear, enables the movement of a finger module.

### Example 3

The modular human hand prosthesis with modular, mechanically independent finger modules as in example 1 except that it is the mechanical and/or hydraulic and or pneumatic gear that causes the rotational movement of the finger distal segment 6 against the finger proximal segment 5.

### Example 4

The modular human hand prosthesis with modular, mechanically independent finger modules as in the example 1 with the exception that it has from two to 4 four mechanically independent finger modules with a load bearing structure and or structures connecting the bases 8 of all modules or groups of modules, replacing the lost fingers group.

### Example 5

The modular human hand prosthesis with modular, mechanically independent finger modules as in the example 2, substantial in that it has from two to 4 four mechanically independent finger modules with the load bearing structure and/or structures connecting the bases 8 of all modules or groups of modules, replacing the lost fingers group.

### Example 6

The modular human hand prosthesis with modular, mechanically independent finger modules as in the example 3 substantial in that it has from two to 4 four mechanically independent finger modules with the load bearing structure and/or structures connecting the bases 8 of all modules or groups of modules, replacing the lost fingers group.

### Example 7

The modular human hand prosthesis with modular, mechanically independent finger modules as in the example 1 substantial in that it has five mechanically independent finger modules with the load bearing structure connecting the bases 8 of all modules forms a design replacing all the lost hand fingers with or without the need to replace the whole hand.

### Example 8

The modular human hand prosthesis with modular, mechanically independent finger modules as in the example 2 substantial in that it has five mechanically independent finger modules with the load bearing structure connecting the bases 8 of all modules forms a design replacing all the lost hand fingers with or without the need to replace the whole hand.

### Example 9

The modular human hand prosthesis with modular, mechanically independent finger modules as in the example 3 substantial in that it has five mechanically independent finger modules with the load bearing structure connecting the bases 8 of all modules forms a design replacing all the lost hand fingers with or without the need to replace the whole hand.

## Claims

1. A modular human hand prosthesis with modular, mechanically independent finger modules that has at least one mechanically independent finger module particularly consisting of a fixed finger base (8), a finger proximal segment (5), a finger distal segment (6), a rod (7) and a rotational drive (11), **characterized in that** the finger base (8), equipped with a rod rotational proximal joint (1) and a rotational joint of a finger proximal segment (2), is connected via a rod proximal joint (1), the rod (7), a rod distal segment (4) and simultaneously via the proximal joint of the finger proximal segment (2), the finger proximal segment (5), a distal joint of the finger proximal segment (3) with the finger distal segment (6), wherein the distal joint of the finger proximal segment (3) constitutes the rotational centre of the drive (11).

2. The modular human hand prosthesis hand with modular, mechanically independent finger modules according to claim 1, **characterised in that** the finger is composed of at least two segments, of which the finger proximal segment (5) is applied as the proximal phalanx and the finger distal segment (6) is used as the distal phalanx.

3. The modular human hand prosthesis with modular, mechanically independent finger modules according to claim 1, **characterized in that** an electric motor (12), coupled permanently to a reduction gear (13) which is connected to a worm gear (9,10), is used as the main rotational drive (11).

4. The modular human hand prosthesis with modular, mechanically independent finger modules according to claim 3, **characterized in that** the electric motor (12) together with the reduction gear (13) is permanently fixed in the finger proximal segment (5) in such a way that the reduction gear output shaft is facing the finger distal segment (6).

5. The modular human hand prosthesis with modular, mechanically independent finger modules according to claim 3 and 4, **characterized in that** the worm wheel (10) is fixed to the finger distal segment (6).

6. The modular human hand prosthesis with modular, mechanically independent finger modules according to claim 4, **characterized in that** the worm (9) geared with the worm wheel (10) is mounted on the reduction gear output shaft (13).

7. The modular human hand prosthesis with modular, mechanically independent finger modules according to claim 4, **characterized in that** the electric motor (12) together with the reduction gear (13) is positioned along the finger proximal segment (5), wherein the motor axis characterized (12) is inclined from the geometrical axis of the finger proximal segment (5) in the range of +- 30[deg.].

8. The modular human hand prosthesis with modular, mechanically independent finger modules according to claim 1, 2, 3, 4, 5, 6 and 7, **characterized in that** at the time the motor is powered (12), the worm (9), mounted on the motor output shaft (12), causes rotation of the worm wheel (10) and the associated finger distal phalanx (6) against the finger proximal phalanx (5) and, in particular, against the distal joint of the finger proximal phalanx (3).

9. The modular human hand prosthesis with modular, mechanically independent finger modules according to claim 1, 2, 3, 4, 5, 6 and 7, **characterized in that** when the finger distal segment (6) moves it causes rotational movement of the distal segment of the finger proximal segment (3) in relation to the distal segment of the rod (4) and the rod (7) in relation to the proximal segment of the rod (1), causing rotational movement of the finger proximal segment (5) in relation to the proximal segment joint of the finger proximal segment (2) of the finger base (8).

10. The modular human hand prosthesis with modular, mechanically independent finger modules according to claim 1, 2, 3, 4, 5, 6 and 7, **characterized in that** when the motor (12) is powered, the finger proximal segment (5) and the finger distal segment (6) perform, relative to the finger base (8), a complex movement in such a way that the finger distal segment (6) approaches or moves away from the finger base (8) and the finger proximal segment (5), while the proximal segment (5) performs a rotational movement relative to the proximal joint of the finger proximal segment (2) of the finger base (8), causing the closing or opening of the finger module.

11. The modular human hand prosthesis with modular, mechanically independent finger modules according to claim 1, **characterized in that** the rotational drive (11) is provided by electric, pneumatic or hydraulic motor which, through a single gear, a set of gears or without any gear enables the movement of a finger module.

12. The modular human hand prosthesis with modular, mechanically independent finger modules according to claim 1, **characterized in that** it is the mechanical and/or hydraulic and/or pneumatic gear that causes rotational movement of the finger distal segment (6) against the finger proximal segment (5).

13. The modular human hand prosthesis with modular, mechanically independent finger modules according to claim 1, **characterized in that** the ratio of the distance of the horizontal part of the finger distal segment (6) comprising joints (3) and (4) against the perpendicular part to it is 0.24a:a, where 'a' is the characteristic dimension of the finger distal segment (6).

14. The modular human hand prosthesis with modular, mechanically independent finger modules according to claim 1, **characterized in that** the ratio of the distance between joints (3) and (4) to the distal segment part (6) perpendicular to the horizontal part containing joints (3) and (4) is 0.07a.

15. The modular human hand prosthesis with modular, mechanically independent finger modules according to claim 1, **characterized in that** the lines formed by joints (1) and (2), as well as joints (3) and (4), are parallel to each another if the mechanically independent finger module is in its initial position.

16. The modular human hand prosthesis with modular, mechanically independent finger modules according to claim 1 and 15, **characterized in that** the ratio of the distances between joints (1) and (2) to the vertical distance between joints (2) and (4) is 0.32b:b, where 'b' is the characteristic dimension of the finger proximal segment (5).

17. The modular human hand prosthesis with modular, mechanically independent finger modules according to claim 1 and 15, **characterized in that** the ratio of the horizontal distance of the joint (2) from the finger distal segment part (6) perpendicular to the horizontal part containing joints (3) and (4), to the vertical distance between joints (2) and (4) is 0.1b:b.

18. The modular human hand prosthesis with modular, mechanically independent finger modules according to claim 1, **characterized in that** the ratio of the characteristic dimension 'a' of the finger distal segment (6) to the characteristic dimension 'b' of the finger proximal segment (5) is 0.9.

19. The modular human hand prosthesis with modular, mechanically independent finger modules according to claim 1, **characterized in that** it has a single mechanically independent finger module with an element connecting the finger module base (8) with the patient's body and replaces the patient's single lost finger.

20. The modular human hand prosthesis with modular, mechanically independent finger modules according to claim 1, **characterized in that** it has from two to four mechanically independent finger modules with the load bearing structure and/or structures connecting the bases (8) of all modules or groups of modules, replacing the lost fingers group.

21. The modular human hand prosthesis with modular, mechanically independent finger modules according to claim 1, **characterized in that** it has five mechanically independent finger modules with the load bearing structure connecting the bases (8) of all modules forms a structure replacing all the lost hand fingers with or without the need to replace the whole hand.

22. The modular human hand prosthesis with modular, mechanically independent finger modules according to claim 1, **characterized in that** it has additional components and/or load bearing especially in the form of wrist connectors, wrist, fastening systems in case of upper limb short amputation, belts, harnesses, and other elements that perform cosmetic and/or protective functions in relation to the mentioned additional components and/or load bearing.

23. The modular human hand prosthesis with modular, mechanically independent finger modules according to claim 1, **characterized in that** the device is controlled by digital and or analog processed electromyographic signals (EMG) and/or mechanomyographic signals (MMG), in particular acoustomyographic and/or accelleromyographic signals, and other biological signals including in particular electrooculographic (EOG) and electroencephalographic (EEG) signals.

24. The modular human hand prosthesis with modular, mechanically independent finger modules according to claim 1, **characterized in that** it has an outer layer and/or layers that perform cosmetic and/or protective functions against external factors.

## Patentansprüche

1. Modulare menschliche Handprothese mit modularen, mechanisch unabhängigen Fingermodulen, die mindestens ein mechanisch unabhängiges Fingermodul aufweist, bestehend aus einer starren Fingerbasis (8), einem proximalen Fingersegment (5), einem distalen Fingersegment (6), einer Stange (7) und einem Drehantrieb (11), **dadurch gekennzeichnet, dass** die Fingerbasis (8), die mit einer proximalen Drehgelenkstange (1) und einem Drehgelenk eines proximalen Fingersegments (2) ausgestattet ist, über eine proximale Gelenkstange (1), die Stange (7), ein distales Stangensegment (4) und gleichzeitig über das proximale Gelenk des proximalen Fingersegments (2), das proximale Fingersegment (5), das distale Gelenk des proximalen Fingersegments (3) mit dem distalen Fingersegment (6) verbunden ist, wobei das distale Gelenk des proximalen Fingersegments (3) das Drehzentrum des Antriebs (11) bildet.

2. Modulare menschliche Handprothese mit modularen, mechanisch unabhängigen Fingermodulen nach Anspruch 1, **dadurch gekennzeichnet, dass** der Finger im Wesentlichen aus mindestens zwei Segmenten zusammengesetzt ist, von denen das proximale Fingersegment (5) als das proximales Fingerglied eingesetzt und das distale Fingersegment (6) als das distale Fingerglied verwendet wird.

3. Modulare menschliche Handprothese mit modularen, mechanisch unabhängigen Fingermodulen nach Anspruch 1, **dadurch gekennzeichnet, dass** im Wesentlichen ein Elektromotor (12), der permanent mit einem Untersetzungsgetriebe (13) gekoppelt ist, welches mit einem Schneckengetriebe (9, 10) verbunden ist, als der Hauptdrehantrieb (11) verwendet wird.

4. Modulare, menschliche Handprothese mit modularen, mechanisch unabhängigen Fingermodulen nach Anspruch 3, **dadurch gekennzeichnet, dass** im Wesentlichen der Elektromotor (12) zusammen mit dem Untersetzungsgetriebe (13) dauerhaft in dem proximalen Fingersegment (5) derart fixiert ist, dass die Ausgangswelle des Untersetzungsgetriebes in Richtung des distalen Fingersegments (6) weist.

5. Modulare menschliche Handprothese mit modularen, mechanisch unabhängigen Fingermodulen nach Anspruch 3 und 4, **dadurch gekennzeichnet, dass** im Wesentlichen das Schneckenrad (10) an dem distalen Fingersegment (6) fixiert ist.

6. Modulare menschliche Handprothese mit modularen, mechanisch unabhängigen Fingermodulen nach Anspruch 4, **dadurch gekennzeichnet, dass** im Wesentlichen das mit dem Schneckenrad (10) verzahnte Schneckengetriebe (9) an der Ausgangswelle (13) des Untersetzungsgetriebes befestigt ist.

7. Modulare menschliche Handprothese mit modularen, mechanisch unabhängigen Fingermodulen nach Anspruch 4, **dadurch gekennzeichnet, dass** im Wesentlichen der Elektromotor (12) zusammen mit dem Reduktionsgetriebe (13) längs des proximalen Fingersegments (5) angeordnet ist, wobei die Motorachse (12) gegenüber der geometrischen Achse des proximalen Fingersegments (5) im Bereich von +/- 30 Grad geneigt ist.

8. Modulare menschliche Handprothese mit modularen, mechanisch unabhängigen Fingermodulen nach Anspruch 1, 2, 3, 4, 5, 6 und 7, **dadurch gekennzeichnet, dass** im Wesentlichen, wenn der Motor eingeschaltet wird (12), das an der Motorausgangswelle (12) befestigte Untersetzungsgetriebe (9) eine Drehung des Schneckenrads (10) und des zugehörigen distalen Fingerglieds (6) gegen das proximale Fingerglied (5) und insbesondere gegen das distale Gelenk des proximalen Fingerglieds (3) bewirkt.

9. Modulare menschliche Handprothese mit modularen, mechanisch unabhängigen Fingermodulen nach Anspruch 1, 2, 3, 4, 5, 6 und 7, **dadurch gekennzeichnet, dass** im Wesentlichen das Bewegen des distalen Fingersegments (6) eine Drehbewegung des distalen Segments des proximalen Fingersegments (3) in Relation zu dem distalen Segment der Stange (4) und der Stange (7) in Relation zu dem proximalen Segment der Stange (1) bewirkt, so dass eine Drehbewegung des proximalen Fingersegments (5) in Relation zu dem proximalen Segmentgelenk des proximalen Fingersegments (2) der Fingerbasis (8) verursacht wird.

10. Modulare menschliche Handprothese mit modularen, mechanisch unabhängigen Fingermodulen nach Anspruch 1, 2, 3, 4, 5, 6 und 7, **dadurch gekennzeichnet, dass** im Wesentlichen das proximale Fingersegment (5) und das distale Fingersegment (6) bei Einschalten des Motors (12) relativ zu der Fingerbasis (8) eine komplexe Bewegung derart ausführen, dass sich das distale Fingersegment (6) der Fingerbasis (8) und dem proximalen Fingersegment (5) nähert oder sich von diesen entfernt, wobei das proximale Segment eine Drehbewegung relativ zu dem proximalen Gelenk des proximalen Fingersegments (2) der Fingerbasis (8) ausführt, so dass ein Schließen oder Öffnen des Fingermoduls bewirkt wird.

11. Modulare menschliche Handprothese mit modularen, mechanisch unabhängigen Fingermodulen nach Anspruch 1, **dadurch gekennzeichnet, dass** im Wesentlichen der Drehantrieb (11) durch einen elektrischen, pneumatischen oder hydraulischen Motor bereitgestellt wird, der die Bewegung eines Fingermoduls durch ein einziges Zahnrad, einen Zahnradsatz oder ohne jegliche Zahnräder ermöglicht.

12. Modulare menschliche Handprothese mit modularen, mechanisch unabhängigen Fingermodulen nach Anspruch 1, **dadurch gekennzeichnet, dass** im Wesentlichen das mechanische und/oder hydraulische und/oder pneumatische Zahnrad die Drehbewegung des distalen Fingersegments (6) gegenüber dem proximalen Fingersegment (5) bewirkt.

13. Modulare menschliche Handprothese mit modularen, mechanisch unabhängigen Fingermodulen nach Anspruch 1, **dadurch gekennzeichnet, dass** im Wesentlichen das Verhältnis des Abstands des horizontalen Teils des distalen Fingersegments (6), umfassend die Gelenke (3) und (4), gegenüber dem senkrechten Teil dazu 0,24a:a beträgt, wobei "a" die charakteristische Dimension des distalen Fingersegments (6) ist.

14. Modulare menschliche Handprothese mit modularen, mechanisch unabhängigen Fingermodulen nach Anspruch 1, **dadurch gekennzeichnet, dass** im Wesentlichen das Verhältnis des Abstands zwischen den Gelenken (3) und (4) zu dem distalen Segmentteil (6), der senkrecht auf dem horizontalen Teil steht, der die Gelenke (3) und (4) enthält, 0,07a beträgt.

15. Modulare menschliche Handprothese mit modularen, mechanisch unabhängigen Fingermodulen nach Anspruch 1, **dadurch gekennzeichnet, dass** im Wesentlichen die von den Gelenken (1) und (2) sowie von den Gelenken (3) und (4) gebildeten Linien parallel zueinander verlaufen, wenn sich das mechanisch unabhängige Fingermodul in seiner Ausgangsposition befindet.

16. Modulare menschliche Handprothese mit modularen, mechanisch unabhängigen Fingermodulen nach Anspruch 1 und 15, **dadurch gekennzeichnet, dass** im Wesentlichen das Verhältnis der Abstände zwischen den Gelenken (1) und (2) zu dem vertikalen Abstand zwischen den Gelenken (2) und (4) 0,32b:b beträgt, wobei "b" die charakteristische Dimension des proximalen Fingersegments (5) ist.

17. Modulare menschliche Handprothese mit modularen, mechanisch unabhängigen Fingermodulen nach Anspruch 1 und 15, **dadurch gekennzeichnet, dass** im Wesentlichen das Verhältnis des horizontalen Abstands des Gelenks (2) von dem distalen Fingersegmentteil (6), der senkrecht zu dem horizontalen Teil steht, der die Gelenke (3) und (4) enthält, zu dem vertikalen Abstand zwischen den Gelenken (2) und (4) 0,1 b:b beträgt.

18. Modulare menschliche Handprothese mit modularen, mechanisch unabhängigen Fingermodulen nach Anspruch 1, **dadurch gekennzeichnet, dass** im Wesentlichen das Verhältnis der charakteristischen Dimension "a" des distalen Fingersegments (6) zu der charakteristischen Dimension "b" des proximalen Fingersegments (5) 0,9 beträgt.

19. Modulare menschliche Handprothese mit modularen, mechanisch unabhängigen Fingermodulen nach Anspruch 1, **dadurch gekennzeichnet, dass** sie im Wesentlichen ein einzelnes, mechanisch unabhängiges Fingermodul mit einem Element aufweist, das die Fingermodulbasis (8) mit dem Körper des Patienten verbindet und einen einzelnen verlorenen Finger des Patienten ersetzt.

20. Modulare menschliche Handprothese mit modularen, mechanisch unabhängigen Fingermodulen nach Anspruch 1, **dadurch gekennzeichnet, dass** sie im Wesentlichen zwei bis vier mechanisch unabhängige Fingermodule mit der lastaufnehmenden Struktur und/oder Strukturen aufweist, die die Basen (8) aller Module oder Gruppen von Modulen verbindet bzw. verbinden und damit die verlorene Fingergruppe ersetzt.

21. Modulare menschliche Handprothese mit modularen, mechanisch unabhängigen Fingermodulen nach Anspruch 1, **dadurch gekennzeichnet, dass** sie im Wesentlichen fünf mechanisch unabhängige Fingermodule aufweist, wobei die lastaufnehmende Struktur, die die Basen (8) aller Module verbindet, eine Struktur bildet, die alle verlorenen Finger einer Hand ersetzt, ohne dass die gesamte Hand ersetzt werden muss oder auch wenn die gesamte Hand ersetzt werden muss.

22. Modulare menschliche Handprothese mit modularen, mechanisch unabhängigen Fingermodulen nach Anspruch 1, **dadurch gekennzeichnet, dass** sie im Wesentlichen zusätzliche Komponenten und/oder Lastaufnahmen aufweisen, insbesondere in Form von Handgelenksanschlüssen, Handgelenk, Befestigungssystemen im Fall einer Kurzamputation oberer Gliedmaßen, Gurte, Gurtzeug und anderer Elemente, die kosmetischen und/oder schützenden Funktionen in Bezug auf die erwähnten zusätzlichen Komponenten und/oder Lastaufnahmen dienen.

23. Modulare menschliche Handprothese mit modularen, mechanisch unabhängigen Fingermodulen nach Anspruch 1, **dadurch gekennzeichnet, dass** die Vorrichtung im Wesentlichen durch digital und/oder analog verarbeitete elektromyografische Signale (EMG) und/oder mechanomyografische Signale (MMG) gesteuert wird, insbesondere durch akustomyografische und/oder accelleromyografische Signale sowie weitere biologische Signale, umfassend insbesondere elektrooculografische (EOG) und elektroencephalografische (EEG) Signale.

24. Modulare menschliche Handprothese mit modularen, mechanisch unabhängigen Fingermodulen nach Anspruch 1, **dadurch gekennzeichnet, dass** sie im Wesentlichen eine äußere Schicht und/oder Schichten aufweist, die kosmetischen und/oder schützenden Funktionen gegen äußere Einflüsse dienen.

## Revendications

1. Prothèse modulaire de main humaine comportant des modules modulaires de doigts mécaniquement indépendants, présentant au moins un module modulaire de doigt mécaniquement indépendant constitué plus particulièrement d'une base de doigt fixe (8), d'un segment proximal de doigt (5), d'un segment distal de doigt (6), d'une tige (7) et d'un entraînement rotatif (11), **caractérisée en ce que** la base de doigt (8), pourvue d'une liaison proximale rotative de tige (1) et d'une liaison rotative de segment proximal de doigt (2), est raccordée, par le biais de la liaison proximale de tige (1), de la tige (7), d'un segment distal de tige (4) et simultanément par le biais de la liaison proximale de segment proximal de doigt (2), du segment proximal de doigt (5), d'une liaison distale du segment proximal de doigt (3), au segment distal de doigt (6), la liaison distale du segment proximal de doigt (3) constituant le centre de rotation de l'entraînement (11).

2. Prothèse modulaire de main humaine comportant des modules modulaires de doigts mécaniquement indépendants selon la revendication 1, **caractérisée en ce que** le doigt se compose d'au moins deux segments, parmi lesquels le segment proximal de doigt (5) est appliqué comme phalange proximale et le segment distal de doigt (6) sert de phalange distale.

3. Prothèse modulaire de main humaine comportant des modules modulaires de doigts mécaniquement indépendants selon la revendication 1, **caractérisée en ce qu**'un moteur électrique (12), couplé de manière permanente à un réducteur (13) qui est raccordé à un engrenage à vis sans fin (9, 10), sert d'entraînement rotatif principal (11).

4. Prothèse modulaire de main humaine comportant des modules modulaires de doigts mécaniquement indépendants selon la revendication 3, **caractérisée en ce que** le moteur électrique (12) associé au réducteur (13) est fixé de manière permanente dans le segment proximal de doigt (5) de telle manière que l'arbre de sortie du réducteur soit en vis-à-vis du segment distal de doigt (6).

5. Prothèse modulaire de main humaine comportant des modules modulaires de doigts mécaniquement indépendants selon la revendication 3 et 4, **caractérisée en ce que** la roue à vis sans fin (10) est fixée au segment distal de doigt (6).

6. Prothèse modulaire de main humaine comportant des modules modulaires de doigts mécaniquement indépendants selon la revendication 4, **caractérisée en ce que** la vis sans fin (9) en prise avec la roue à vis sans fin (10) est montée sur l'arbre de sortie du réducteur (13).

7. Prothèse modulaire de main humaine comportant des modules modulaires de doigts mécaniquement indépendants selon la revendication 4, **caractérisée en ce que** le moteur électrique (12) associé au réducteur (13) est disposé le long du segment proximal de doigt (5), l'axe du moteur (12) étant incliné par rapport à l'axe géométrique du segment proximal de doigt (5) dans une fourchette de ± 30°.

8. Prothèse modulaire de main humaine comportant des modules modulaires de doigts mécaniquement indépendants selon la revendication 1, 2, 3, 4, 5, 6 et 7, **caractérisée en ce que,** au moment où le moteur est alimenté (12), la vis sans fin (9), montée sur l'arbre de sortie du moteur (12), entraîne la rotation de la roue à vis sans fin (10) et de la phalange distale (6) du doigt associé contre la phalange proximale de doigt (5) et, plus particulièrement, contre la liaison distale de la phalange proximale de doigt (3).

9. Prothèse modulaire de main humaine comportant des modules modulaires de doigts mécaniquement indépendants selon la revendication 1, 2, 3, 4, 5, 6 et 7, **caractérisée en ce que,** lorsque le segment distal de doigt (6) effectue un mouvement, il entraîne le mouvement de rotation du segment distal du segment proximal de doigt (3) relativement au segment distal de tige (4) et de la tige (7) relativement au segment proximal de tige (1), entraînant le mouvement de rotation du segment proximal de doigt (5) relativement à la liaison de segment proximal du segment proximal de doigt (2) de la base de doigt (8).

10. Prothèse modulaire de main humaine comportant des modules modulaires de doigts mécaniquement indépendants selon la revendication 1, 2, 3, 4, 5, 6 et 7, **caractérisée en ce que,** lorsque le moteur (12) est alimenté, le segment proximal de doigt (5) et le segment distal de doigt (6) effectuent, relativement à la base de doigt (8), un mouvement complexe selon lequel le segment distal de doigt (6) s'approche ou s'éloigne de la base de doigt (8) et du segment proximal de doigt (5), tandis que le segment proximal (5) effectue un mouvement de rotation par rapport à la liaison proximale du segment proximal de doigt (2) de la base de doigt (8), entraînant la fermeture ou l'ouverture du module de doigt.

11. Prothèse modulaire de main humaine comportant des modules modulaires de doigts mécaniquement indépendants selon la revendication 1, **caractérisée en ce que** l'entraînement rotatif (11) est assuré par un moteur électrique, pneumatique ou hydraulique qui, par le biais d'un seul engrenage, d'un ensemble d'engrenages ou sans aucun engrenage, permet le mouvement d'un module de doigt.

12. Prothèse modulaire de main humaine comportant des modules modulaires de doigts mécaniquement indépendants selon la revendication 1, **caractérisée en ce que** c'est un engrenage mécanique et/ou hydraulique et/ou pneumatique qui entraîne le mouvement de rotation du segment distal de doigt (6) contre le segment proximal de doigt (5).

13. Prothèse modulaire de main humaine comportant des modules modulaires de doigts mécaniquement indépendants selon la revendication 1, **caractérisée en ce que** le rapport existant entre la longueur de la partie horizontale du segment distal de doigt (6), comprenant les liaisons (3) et (4), et celle de la partie qui y est perpendiculaire est de 0,24a:a, où « a » représente la dimension caractéristique du segment distal de doigt (6).

14. Prothèse modulaire de main humaine comportant des modules modulaires de doigts mécaniquement indépendants selon la revendication 1, **caractérisée en ce que** le rapport existant entre la distance séparant les liaisons (3) et (4) et la partie de segment distal (6) perpendiculaire à la partie horizontale contenant les liaisons (3) et (4) est de 0,07a.

15. Prothèse modulaire de main humaine comportant des modules modulaires de doigts mécaniquement indépendants selon la revendication 1, **caractérisée en ce que** les lignes formées par les liaisons (1) et (2), ainsi que les liaisons (3) et (4), sont parallèles entre elles si le module de doigt mécaniquement indépendant est dans sa position initiale.

16. Prothèse modulaire de main humaine comportant des modules modulaires de doigts mécaniquement indépendants selon la revendication 1 et 15, **caractérisée en ce que** le rapport existant entre les distances séparant les liaisons (1) et (2) et la distance verticale séparant les liaisons (2) et (4) est de 0,32b:b, où « b » représente la dimension caractéristique du segment proximal de doigt (5).

17. Prothèse modulaire de main humaine comportant des modules modulaires de doigts mécaniquement indépendants selon la revendication 1 et 15, **caractérisée en ce que** le rapport existant entre la distance horizontale séparant la liaison (2) et la partie de segment distal de doigt (6) perpendiculaire à la partie horizontale contenant les liaisons (3) et (4) et la distance verticale séparant les liaisons (2) et (4) est de 0,1 b:b.

18. Prothèse modulaire de main humaine comportant des modules modulaires de doigts mécaniquement indépendants selon la revendication 1, **caractérisée en ce que** le rapport existant entre la dimension caractéristique « a » du segment distal de doigt (6) et la dimension caractéristique « b » du segment proximal de doigt (5) est de 0,9.

19. Prothèse modulaire de main humaine comportant des modules modulaires de doigts mécaniquement indépendants selon la revendication 1, **caractérisée en ce qu**'elle présente un seul module de doigt mécaniquement indépendant comportant un élément reliant la base du module de doigt (8) au corps du patient et en ce qu'elle remplace ledit doigt perdu du patient.

20. Prothèse modulaire de main humaine comportant des modules modulaires de doigts mécaniquement indépendants selon la revendication 1, **caractérisée en ce qu**'elle présente de deux à quatre modules modulaires de doigts mécaniquement indépendants, la ou les structures destinées à supporter une charge reliant les bases (8) de tous les modules ou groupes de modules, en remplacement du groupe de doigts perdus.

21. Prothèse modulaire de main humaine comportant des modules modulaires de doigts mécaniquement indépendants selon la revendication 1, **caractérisée en ce qu**'elle présente cinq modules de doigts mécaniquement indépendants, la structure destinée à supporter une charge reliant les bases (8) de tous les modules, en formant une structure remplaçant tous les doigts de la main perdus sans nécessairement devoir remplacer la totalité de la main.

22. Prothèse modulaire de main humaine comportant des modules modulaires de doigts mécaniquement indépendants selon la revendication 1, **caractérisée en ce qu**'elle présente des composants et/ou structures supplémentaires destinés à supporter une charge, notamment sous la forme de connecteurs de poignet, poignet, systèmes de fixation dans le cas d'amputations courtes du membre supérieur, de courroies, harnais et autres éléments assurant des fonctions esthétiques et/ou protectrices en lien avec lesdits composants et/ou structures supplémentaires destinés à supporter une charge.

23. Prothèse modulaire de main humaine comportant des modules modulaires de doigts mécaniquement indépendants selon la revendication 1, **caractérisée en ce que** le dispositif est commandé par des signaux électromyographiques (EMG) et/ou mécanomyographiques (MMG) à traitement numérique et/ou analogique, notamment des signaux acoustomyographiques et/ou accéléromyographiques, et d'autres signaux biologiques parmi lesquels notamment les signaux électrooculographiques (EOG) et électroencéphalographiques (EEG).

24. Prothèse modulaire de main humaine comportant des modules modulaires de doigts mécaniquement indépendants selon la revendication 1, **caractérisée en ce qu**'elle présente une ou des couches extérieures qui assurent des fonctions esthétiques et/ou protectrices vis-à-vis de facteurs extérieurs.
